# EUROPÄISCHE PATENTSCHRIFT

(11) **EP 0 735 115 B1**
(45) Veröffentlichungstag und Bekanntmachung des Hinweises auf die Patenterteilung: **28.02.2001**
(21) Anmeldenummer: 96104563.0
(22) Anmeldetag: 22.03.1996
(51) Int. Cl.: C09C 1/00, C09D 7/12, C09D 11/00, C08K 9/02, C04B 33/14, C03C 4/02, A61K 7/00

(54) **Verfahren zur Herstellung von blaustichigen Glanzpigmenten**
Process for preparing bluish brilliant pigments
Procédé de préparation de pigments brillants bleuâtres

(30) Priorität: 30.03.1995 DE 19511697
(43) Veröffentlichungstag der Anmeldung: 02.10.1996
(73) Patentinhaber: BASF Aktiengesellschaft, 67063 Ludwigshafen (DE)
(72) Erfinder: Schmidt, Helmut, 67574 Osthofen (DE); Ostertag, Werner, Dr., 67269 Grünstadt (DE); Bidlingmaier, Hermann, 77654 Offenburg (DE); Mronga, Norbert, Dr., 69221 Dossenheim (DE); Gonzalez Gomez, Juan Antonio, Dr., 67063 Ludwigshafen (DE); Kaliba, Claus, Dr., 67141 Neuhofen (DE); Schmid, Raimund, Dr., 67435 Neustadt (DE); Ellinghoven, Raymond, 71672 Marbach (DE)

(56) Entgegenhaltungen:
- EP-A- 0 332 071
- PATENT ABSTRACTS OF JAPAN vol. 9, no. 78 (C-274), 6.April 1985 & JP-A-59 212422 (SHISEIDO), 1.Dezember 1984,
- PATENT ABSTRACTS OF JAPAN vol. 10, no. 31 (C-327), 6.Februar 1986 & JP-A-60 184570 (SHISEIDO), 20.September 1985,
- DATABASE WPI Week 8641 Derwent Publications Ltd., London, GB; AN 86-267354 XP002006649 & JP-A-61 192 749 (HINODE KAGAKU KOGYO) , 27.August 1986 & JP-A-61 192 749 (...)

## Beschreibung

Die vorliegende Erfindung betrifft ein neues Verfahren zur Herstellung von blaustichigen Glanzpigmenten durch Behandlung titandioxidbeschichteter silikatischer Plättchen mit einem reduzierenden Gasgemisch.

Außerdem betrifft die Erfindung neue blaustichige Glanzpigmente auf Basis silikatischer Plättchen, die eine reduzierte Titandioxidbeschichtung mit einer geometrischen Schichtdicke von 10 bis 60 nm und CIELAB-Farbwerte -2 ≤ a* < 2 und b* ≤ - 20 (Meßwinkel 20°, Normlichtart D65) aufweisen, sowie deren Verwendung zum Einfärben von Lacken, Druckfarben, Tinten, Kunststoffen, Gläsern, keramischen Produkten und Zubereitungen der dekorativen Kosmetik.

Reduzierte titandioxidbeschichtete Glimmerpigmente, deren TiO₂-Beschichtung reduzierte Titanspezies (Oxidationszahl des Titans < 4 bis 2) enthält oder gänzlich in diese reduzierten Spezies umgewandelt ist, sind schon länger als "dunkle Perlglanzpigmente" für den blauen bis schwarzen Farbtonbereich bekannt und zeichnen sich durch gutes Deckvermögen, Farbstärke und Glanz aus.

Die dunklen Perlglanzpigmente lassen sich bekanntermaßen durch Behandlung von titandioxidbeschichteten Glimmerpigmenten mit reduzierenden Gasen bei Temperaturen von 600 bis 900°C herstellen.

Als reduzierende Gase werden dabei neben Propan, bei dessen Anwendung nicht nur reduzierte Titanspezies, sondern auch Kohlenstoff enthaltende Produkte anfallen (JP-A-192 749/1985, EP-A-525 526), und Wasserstoff (JP-A-192 749/1985) vorwiegend Ammoniak und Ammoniak/Stickstoff-Gemische eingesetzt. Die erhaltenen Produkte werden als blau, blauschwarz oder schwarz und die reduzierten Spezies als niedere Titanoxide wie Ti₃O₅, Ti₂O₃ bis zu TiO, Titanoxynitride sowie Titannitrid beschrieben (JP-A-164 653/1983, JP-A-126 468/1984, JP-A-184 570/1985 und DE-A-34 33 657).

Aus der EP-A-332 071 ist schließlich auch ein Verfahren zur Herstellung besonders blaustichiger Perlglanzpigmente bekannt, bei dem silberne oder blau reflektierende TiO₂-beschichtete Glimmer (optische TiO₂-Schichtdicke 50 bis 100 nm bzw. 300 bis 340 nm) unter ständiger Bewegung bei 750 bis 850°C mit Ammoniak reduziert werden.

Ganz besonders blaustichige und gleichzeitig farbtonreine Pigmente werden hier jedoch nur durch Reduktion der teuren, blau reflektierenden Glimmer mit hoher TiO₂-Schichtdicke erhalten, während durch Behandlung der kostengünstigeren silbernen Glimmer mit geringer TiO2-Schichtdicke auch nach langen Behandlungszeiten Blautöne resultieren, die zwar farbintensiv (b* ≈ -20) sind, aber einen hohen Grünanteil (a* ≈ -5) aufweisen.

Der Erfindung lag daher die Aufgabe zugrunde, die Herstellung besonders blaustichiger, farbtonreiner Perlglanzpigmente auf kostengünstige, reproduzierbare Weise zu ermöglichen.

Demgemäß wurde ein Verfahren zur Herstellung von blaustichigen Glanzpigmenten durch Behandlung titandioxidbeschichteter silikatischer Plättchen mit einem reduzierenden Gasgemisch gefunden, welches dadurch gekennzeichnet ist, daß man titandioxidbeschichtete silikatische Plättchen, deren Titandioxidbelegung eine geometrische Schichtdicke von 10 bis 60 nm aufweist, bei 800 bis 900°C mit einem Gasgemisch, das eine verdampfte organische Verbindung und Ammoniak enthält, behandelt.

Außerdem wurden die eingangs definierten blaustichigen Glanzpigmente und ihre Verwendung zur Einfärbung von Lacken, Druckfarben, Tinten, Kunststoffen, Gläsern, keramischen Produkten und zubereitungen der dekorativen Kosmetik gefunden.

Für die erfindungsgemäßen blaustichigen Glanzpigmente eignen sich als plättchenförmige silikatische Substratmaterialien insbesondere helle oder weiße Glimmer, wobei Schuppen von vorzugsweise naß vermahlenem Muskovit besonders bevorzugt sind. Selbstverständlich sind auch andere natürliche Glimmer, wie Phlogopit oder Biotit, künstliche Glimmer, Talk- und Glasschuppen geeignet.

Die Substratteilchen sind mit einer im wesentlichen aus Titandioxid bestehenden Schicht belegt, die als untergeordnete Bestandteile (im allgemeinen < 5 Gew.-%) weitere, vorzugsweise farblose, Metalloxide wie Zirkondioxid, Zinndioxid, Aluminiumoxid und Siliciumdioxid enthalten kann, und eine geometrische Schichtdicke von in der Regel 10 bis 60 nm, bevorzugt 20 bis 40 nm, aufweist.

Diese silbernen Perlglanzpigmente sind allgemein bekannt und beispielsweise in den DE-A-14 67 468, DE-A-32 37 264 oder DE-A-20 09 566 beschrieben sowie unter dem Namen Iriodin® (E. Merck, Darmstadt), Flonac® (Kemira Oy, Pori, Finnland) oder Mearlin® (Mearl Corporation, Ossining, New York) im Handel.

Selbstverständlich könnte man beim erfindungsgemäßen Verfahren auch Perlglanzpigmente mit blauer Interferenzfarbe als Ausgangsmaterial einsetzen, die silbernen Pigmente sind aber aus Kostengründen vorzuziehen.

Die Größe der Substratteilchen ist an sich nicht kritisch und kann auf den jeweiligen Anwendungszweck abgestimmt werden. In der Regel haben die plättchenförmigen Teilchen mittlere größte Durchmesser von etwa 1 bis 200 µm, insbesondere etwa 5 bis 100 µm, und Dicken von etwa 0,1 bis 1 µm, insbesondere um etwa 0,5 µm. Ihre spezifische freie Oberfläche (BET) liegt üblicherweise bei 1 bis 15 m²/g, insbesondere 3 bis 12 m²/g.

Beim erfindungsgemäßen Verfahren zur Herstellung der blaustichigen Glanzpigmente werden die silbernen titandioxidbeschichteten silikatischen Plättchen bei in der Regel 800 bis 900°C, bevorzugt 820 bis 880°C, mit einem Gasgemisch behandelt, das eine verdampfte organische Verbindung und Ammoniak enthält.

Geeignete organische Verbindungen sind sowohl gasförmige Verbindungen als auch solche Verbindungen, die bei Raumtemperatur flüssig oder auch fest sind und verdampft werden können.

Bevorzugt sind dabei Kohlenwasserstoffe, besonders gesättigte aliphatische C₁-C₈-Kohlenwasserstoffe wie Pentan, Isopentan, Neopentan, Hexan, Heptan und Octan sowie deren verzweigte Isomere und ganz besonders Methan, Ethan, Propan und Butan und deren Isomere, auch ungesättigte aliphatische C₂-C₄-Kohlenwasserstoffe wie Ethen, Propen, Buten und deren Isomere und aromatische Kohlenwasserstoffe wie Benzol und Toluol.

Selbstverständlich können auch Gemische dieser Kohlenwasserstoffe, wie sie zum Beispiel im Erdgas vorliegen, eingesetzt werden.

Es können auch solche organischen Verbindungen zum Einsatz kommen, die nicht nur Kohlenstoff und Wasserstoff, sondern auch Sauerstoff und/oder Stickstoff enthalten, wobei jedoch je Kohlenstoffatom nicht mehr als ein Sauerstoff- und/oder Stickstoffatom im Molekül vorliegen sollte.

Beispiele für geeignete Verbindungen dieser Art sind C₁-C₅-Alkohole wie Methanol, Ethanol, Propanol und Isopropanol, C₃-C₇-Ketone wie Aceton, Di-C₁-C₂-alkylether wie Dimethyl- und Diethylether und cyclische Ether wie Tetrahydrofuran sowie Mono-C₁-C₅-alkylamine und Monoarylamine wie Methylamin, Ethylamin, Propylamin und Anilin, die auch allein, ohne Ammoniak eingesetzt werden könnten, wenn das Kohlenstoff/Stickstoff-Verhältnis der gewünschten Zusammensetzung des reduzierenden Gasgemisches entspricht. In der Regel sind die Amine jedoch schwerer zu handhaben.

Das in dem reduzierenden Gasgemisch vorliegende Volumenverhältnis der bevorzugten Komponenten Ammoniak und Kohlenwasserstoff kann stark variiert werden und beträgt in der Regel 99:1 bis 50:50, wobei insbesondere bei Verwendung von Methan, Ethan und/oder Propan ein Volumenverhältnis von 95:5 bis 70:30 bevorzugt ist.

Zweckmäßigerweise wird das reduzierende Gasgemisch durch ein inertes Gas wie Stickstoff verdünnt. Dies empfiehlt sich insbesondere dann, wenn die organische Verbindung erst verdampft werden muß und mit Hilfe des inerten Gasstromes vorteilhaft in den Reaktionsraum überführt wird. Besonders günstig ist ein Stickstoffgehalt von 10 bis 60 Vol.-%, bezogen auf die gesamte Gasmenge. Man kann jedoch auch mit einem nur aus Kohlenwasserstoff und Ammoniak bestehenden Gasgemisch arbeiten.

Durch die Einwirkung der organische Verbindung (besonders Kohlenwasserstoff) und Ammoniak enthaltenden Gasgemische wird auch im erfindungsgemäßen Fall (zumindest) teilweise die TiO₂-Schicht des Pigments reduziert, wobei die eingangs beschriebenen reduzierten Spezies entstehen. Der Reduktionsgrad wird mit zunehmender Reaktionstemperatur und zunehmender Reaktionszeit größer, parallel dazu steigt auch der Stickstoffgehalt der Glanzpigmente, d.h. der Anteil an nitridischen Titanverbindungen.

In der Regel weisen die erhaltenen Glanzpigmente Stickstoffgehalte von 0,2 bis 3 Gew.-%, bevorzugt 0,2 bis 2 Gew.-%, auf.

Überraschenderweise werden erfindungsgemäß, also bei Reduktion mit Kohlenwasserstoff/Ammoniak-Gemischen, nur sehr geringe Mengen Kohlenstoff in die Glanzpigmente (bzw. in deren reduzierte Titandioxidbeschichtung) eingebaut, die in der Regel nur 1 bis 20 Gew.-% der bei analog durchgeführter Reduktion unter alleiniger Verwendung des Kohlenwasserstoffs eingebauten Kohlenstoffmenge betragen.

Daher werden besonders blaustichige und gleichzeitig farbtonreine Glanzpigmente erhalten, die im allgemeinen CIELAB-Werte -2 ≤ a* ≤ 2 und b* ≤ -20 aufweisen (Meßwinkel 20°, Normlichtart D65).

Die erfindungsgemäße Reduktion dauert in der Regel 1 bis 4 h, insbesondere 2 bis 3 h, und erfolgt damit deutlich schneller als die Reduktion bei ausschließlicher Verwendung von Ammoniak.

Das erfindungsgemäße Verfahren kann sowohl kontinuierlich, z.B. in einem beheizten, inertisierten Drehrohrofen, in den das Glimmerpigment eingetragen und die reduzierenden Gase im Gemisch mit einem Inertgas eingeleitet werden, als auch diskontinuierlich, z.B. in einer beheizten, inertisierten Drehtrommel mit Gaszu- und -ableitung oder einem beheizten, inertisierten Wirbelbettreaktor, durchgeführt werden, wobei zweckmäßigerweise für eine allseitige Umspülung der Glimmerplättchen mit dem Reduktionsgas gesorgt wird, was im Drehrohrofen oder in der Drehtrommel vorzugsweise durch Stolperleisten ermöglicht wird.

Nach Beendigung der Reduktion wird das Glanzpigment vorzugsweise unter Inertgas abgekühlt. Gewünschtenfalls kann ein Desagglomerierungsschritt, beispielsweise in einem mit Schlagmessern ausgerüsteten Mischer, angeschlossen werden.

Mit Hilfe des erfindungsgemäßen Verfahrens können besonders blaustichige (und farbtonreine) Glanzpigmente auf wirtschaftliche Weise (kostengünstige Ausgangsmaterialien, kurze Behandlungszeiten) reproduzierbar hergestellt werden.

Die erfindungsgemäßen Glanzpigmente eignen sich vorteilhaft für viele Zwecke, wie zur Einfärbung von Kunststoffen, Gläsern, keramischen Produkten, Zubereitungen der dekorativen Kosmetik und insbesondere von Lacken, Tinten und Druckfarben. Bei der Applikation im Druck sind alle industrieüblichen Druckverfahren, z.B. Siebdruck, Tiefdruck, Bronzierdruck, Flexodruck und Offsetdruck, geeignet.

Für diese Anwendungszwecke lassen sich die erfindungsgemäßen Glanzpigmente auch vorteilhaft in Abmischung mit transparenten und deckenden Weiß-, Bunt- und Schwarzpigmenten sowie auch herkömmlichen Glanzpigmenten verwenden.

Als Beispiele für geeignete Glanzpigmente seien ein- oder mehrfach metalloxidbeschichtete Glimmer- und Metallpigmente, unbeschichtete Metallpigmente auf Aluminiumbasis, schwarze Glanzpigmente, wie plättchenförmige Graphit- und Magnetitpigmente, und plättchenförmige Metalloxidpigmente, z.B. auf Basis von Eisen(III)oxid und Bismutoxychlorid, genannt.

Geeignete anorganische Pigmente sind z.B. Titanoxide, dotierte Titanoxide wie Nickeltitangelb, Eisenoxide, Bismutvanadate, farbige Spinelle, Chromat- und Cadmiumpigmente.

Als organische Pigmente eignen sich beispielsweise Monoazopigmente (z.B. Produkte, die sich von Acetessigarylidderivaten oder von β-Naphtholderivaten ableiten), verlackte Monoazofarbstoffe wie verlackte β-Hydroxynaphthoesäurefarbstoffe, Disazopigmente, kondensierte Disazopigmente, Isoindolinderivate, Derivate der Naphthalindicarbonsäure, Derivate der Perylentetracarbonsäure, Anthrachinonpigmente, Thioindigoderivate, Azomethinderivate, Chinacridone, Dioxazine, Pyrazolochinazolone, Indanthron- und Phthalocyaninpigmente.

### Beispiele

### Herstellung und Beurteilung von erfindungsgemäßen Glanzpigmenten

Die Herstellung der Glanzpigmente erfolgte in einem über einen Motor drehbaren und von einem zweischaligen Klappofen beheizten Einhalsrundkolben aus Quarzglas mit einer Gaszu- und -ableitung in der Drehachse.

Zur Beurteilung der Koloristik der Pigmente im Lack wurden je 4 g der Pigmentproben in 96 g eines Polyester-Mischlackes mit 21 Gew.-% Festkörpergehalt eingerührt und 15 min unter 1500 Upm mit einem Propellerrührer dispergiert. Danach wurde der Basislackansatz auf eine Spritzviskosität von 18 sec im DIN-Becher 4 (DIN 53 211) eingestellt und auf ein nicht grundiertes Aluminiumblech gespritzt. Nach einer kurzen Ablüftzeit wurde naß in naß mit einem Einkomponenten-Klarlack auf Basis Acrylat/Melaminharz (47 Gew.-% Festkörper, eingestellt auf 23 Sec-DIN 4) überlackiert. Nach 30minütigem Ablüften bei Raumtemperatur wurde 30 min bei 130°C eingebrannt.

Die Messung der CIELAB-Werte erfolgte anschließend mit einem Gonio-Spektralphotometer Multiflash M45 der Fa. Optronik (Berlin) bei einer Winkeldifferenz von 20°, 45° bzw. 75° zum Glanzwinkel. Die in der Tabelle angegebenen Farbwerte (L, a*, b*, HGD, C*) beziehen sich auf die Normlichtart D65. Dabei entspricht L der Helligkeit, a* dem Rot- bzw. Grünanteil und b* dem Blau- bzw. Gelbanteil. HGD ist der Farbwinkel [°] und C* das Chroma.

### Beispiel 1

75 g eines silbernen titandioxidbeschichteten Glimmerpigments (geometrische TiO₂-Schichtdicke etwa 30 nm; Iriodin® 103 Rutil Sterling Silber; E. Merck, Darmstadt) wurden in der oben beschriebenen Apparatur durch einstündiges Überleiten von 75 l/h Stickstoff inertisiert. Nach Erhitzen auf 850°C wurde 2 h ein Gemisch aus 21 l/h Ammoniak, 6 l/h Ethan und 10 l/h Stickstoff durch die Apparatur geleitet. Das anschließende Abkühlen auf Raumtemperatur erfolgte unter 30 l/h Stickstoff.

Es wurde ein intensiv blau gefärbtes Pigment mit einem Stickstoffgehalt von 1,5 Gew.-% und einem Kohlenstoffgehalt von 0,06 Gew.-% erhalten.

### Vergleichsbeispiel V1

Es wurde analog Beispiel 1 vorgegangen, jedoch wurde ein lediglich aus 21 l/h Ammoniak und 16 l/h Stickstoff bestehendes Gasgemisch verwendet.

Das erhaltene Pigment war deutlich schwächer und grünstichiger blau und hatte einen Stickstoffgehalt von 0,26 Gew.-%.

### Vergleichsbeispiel V2

Es wurde analog Beispiel 1 vorgegangen, jedoch wurde ein lediglich aus 6 l/h Ethan und 31 l/h Stickstoff bestehendes Gasgemisch verwendet.

Das erhaltene Pigment zeigte anstelle der weißen eine silberne Körperfarbe und hatte einen Kohlenstoffgehalt von 4,8 Gew.-%.

**Tabelle:**

| Koloristische Daten | | | | | |
|---|---|---|---|---|---|
| Beispiel | HGD | C* | L | a* | b* |
| | | | | | |

| Meßwinkel 20° | | | | | |
|---|---|---|---|---|---|
| 1 | 268,9 | 21,8 | 66,7 | -0,4 | -21,8 |
| V1 | 239,8 | 11,2 | 94,4 | -5,6 | - 9,6 |
| V2 | 130,3 | 2,9 | 74,4 | -1,9 | 2,2 |

| Meßwinkel 45° | | | | | |
|---|---|---|---|---|---|
| 1 | 268,4 | 11,4 | 19,3 | -0,3 | -11,4 |
| V1 | 245,4 | 7,8 | 35,9 | -3,2 | - 7,0 |
| V2 | 167,4 | 0,8 | 24,9 | -0,8 | 2,2 |

| Meßwinkel 75° | | | | | |
|---|---|---|---|---|---|
| 1 | 267,8 | 8,0 | 7,1 | -0,3 | - 0,8 |
| V1 | 254,5 | 7,7 | 18,9 | -2,0 | - 7,5 |
| V2 | 303,9 | 1,0 | 12,8 | -0,3 | - 1,0 |

## Patentansprüche

1. Verfahren zur Herstellung von blaustichigen Glanzpigmenten durch Behandlung titandioxidbeschichteter silikatischer Plättchen mit einem reduzierenden Gasgemisch, dadurch gekennzeichnet, daß man titandioxidbeschichtete silikatische Plättchen, deren Titandioxidbelegung eine geometrische Schichtdicke von 10 bis 60 nm aufweist, bei 800 bis 900°C mit einem Gasgemisch, das eine verdampfte organische Verbindung und Ammoniak enthält, behandelt.

2. Verfahren nach Anspruch 1, dadurch gekennzeichnet, daß man als organische Verbindung Methan, Ethan, Propan, n-Butan und/oder iso-Butan einsetzt.

3. Blaustichige Glanzpigmente auf Basis silikatischer Plättchen, die eine reduzierte Titandioxidbeschichtung mit einer geometrischen Schichtdicke von 10 bis 60 nm und CIELAB-Farbwerte -2 ≤ a* ≤ 2 und b* ≤ -20 (Meßwinkel 20°, Normlichtart D65) aufweisen.

4. Blaustichige Glanzpigmente nach Anspruch 3, bei denen die silikatischen Plättchen Glimmerplättchen sind.

5. Verwendung von blaustichigen Glanzpigmenten gemäß Anspruch 3 oder 4 zur Einfärbung von Lacken, Druckfarben, Tinten, Kunststoffen, Gläsern, keramischen Produkten und Zubereitungen der dekorativen Kosmetik.

## Claims

1. A process for producing bluish luster pigments by treating titania-coated silicatic platelets with a reducing gas mixture, which comprises treating titania-coated silicatic platelets whose titania coating has a geometric layer thickness of from 10 to 60 nm at from 800 to 900°C with a gas mixture comprising a vaporized organic compound and ammonia.

2. A process as claimed in claim 1, wherein the organic compound used is methane, ethane, propane, n-butane and/or isobutane.

3. Bluish luster pigments based on silicatic platelets comprising a reduced titania coating with a geometric layer thickness of from 10 to 60 nm and CIELAB color coordinates -2 ≤ a* ≤ 2 and b* ≤ -20 (measuring angle 20°, standard illuminant D65).

4. Bluish luster pigments as claimed in claim 3, wherein the silicatic platelets are mica platelets.

5. The use of bluish luster pigments as claimed in claim 3 or 4 for coloring paints, inks, including printing inks, plastics, glasses, ceramic products and decorative cosmetic preparations.

## Revendications

1. Procédé de préparation de pigments brillants à dominante bleue par traitement de plaquettes à base de silicate revêtues de dioxyde de titane avec un mélange gazeux réducteur, caractérisé en ce que l'on traite des plaquettes à base de silicate revêtues de dioxyde de titane dont la couche de dioxyde de titane présente une épaisseur de couche géométrique de 10 à 60 nm, à une température de 800°C à 900°C, avec un mélange gazeux qui contient un composé organique vaporisé et de l'ammoniac.

2. Procédé selon la revendication 1, caractérisé en ce que l'on met en oeuvre le méthane, l'éthane, le propane, le n-butane et/ou l'isobutane en tant que composé organique.

3. Pigments brillants à dominante bleue à base de plaquettes de silicate, qui présentent un revêtement réduit de dioxyde de titane ayant une épaisseur de couche géométrique de 10 à 60 nm et des valeurs chromatiques CIELAB, -2 ≤ a* ≤ 2 et b* ≤ -20 (angle de mesure 20°, lumière d'étalon colorimétrique D65).

4. Pigments brillants à dominante bleue selon la revendication 3, dans lesquels les plaquettes de silicate sont des plaquettes de mica.

5. Utilisation de pigments brillants à dominante bleue selon la revendication 3 ou 4 pour la coloration de laques, d'encres d'impression, d'encres, de matières plastiques, de verres, de produits céramiques et de préparations de la cosmétique décorative.
